# EUROPEAN PATENT APPLICATION

(11) **EP 1 752 154 A1**
(43) Date of publication of application: **14.02.2007**
(21) Application number: 04745418.6
(22) Date of filing: 28.05.2004
(51) Int. Cl.: A61K 36/00

(54) **COMPOSITION COMPRISING FERMENTED TEA EXTRACT AS THE MAIN COMPONENT AND HAVING EFFECT OF INCRASING ADIPONECTIN**

(71) Applicant: SUNTORY LIMITED, Osaka-shi, Osaka 530-8203 (JP)
(72) Inventor: SHIMADA, Kenei, Takaishi-shi, Osaka 5920014 (JP); YOSHIKAWA, Junichi, Kobe-shi, Hyogo 6580073 (JP)
(74) Representative: Vossius & Partner
(86) International application number: PCT/JP2004/007382
(87) International publication number: WO 2005/115425

(57) **Abstract**

A composition capable of elevating blood adiponectin level and enlarging LDL particle size and thus lowering the concentration of small dense LDL in blood to thereby prevent diabetes and a heart disease such as a coronary artery disease or ameliorate the symptoms of these diseases, which originates in a natural material, shows a high safety without any side effect even in continuous intake over a long period of time, and is usable as a health food or drink. A composition comprising as the active ingredient a fermented tea extract which has an effect of elevating adiponectin level and an effect of enlarging LDL particle size.

## Description

### TECHNICAL FIELD

This invention relates to a composition capable of elevating blood adiponectin level and enlarging LDL particle size in the body (in particular, in blood), which comprises as the active ingredient a fermented tea extract and prevents diabetes and a heart disease such as a coronary artery disease or ameliorates the symptoms of these diseases.

### BACKGROUND ART

In these days, deaths due to heart diseases and cerebrovascular diseases become the major causes of death and amount to about 1/3 of the total cases in Japan. These diseases are still increasing year by year. To avoid the onset of these ischemic diseases, it is important to prevent diseases such as hypertension, hyperlipemia and diabetes, which serve as risk factors thereof, by changing life style habits, for example, smoking and lack of exercise. In a patient already suffering from a coronary artery disease such as old myocardial infarction or stable angina pectoris, secondary prevention, i.e., the prevention of recurrence of the disease is an important problem too.

It has been considered hitherto that adipose tissue merely serves as an energy storage container from which excessive energy having been stored therein is supplied when necessary. In the course of studying the clinical conditions of metabolic disorder syndromes typified by life style-related diseases, however, the importance of adipose tissue has been highlighted in recent years. That is, adipose tissue is the largest endocrine organ in vivo which secretes adipose tissue-origin endocrine factors such as plasminogen activator inhibitor-1 (PAI-1), tumor necrosis factor (TNF-α), leptin and adiponectin. Usually, these adipose tissue-origin endocrine factors contribute to the maintenance of homeostasis *in vivo.* In the state with adipose accumulation, however, it has been clarified that these factors are excessively or insufficiently produced or secreted and such disruption of the balance closely relates to the onset and progress of sugar and lipid metabolic errors, hypertension and arteriosclerosis (see, for example, Non-Patent Document 1).

Among these adipose tissue-origin endocrine factors, adiponectin is a 30 kDa secretory protein that is overexpressed in adipose cells and is specific to adipose tissue. In human blood, the concentrations of PIA-1, TNF-α and leptin are increased with the progress of obesity and adipose accumulation. In contrast, blood adiponectin level is lowered in obese persons and elevated by weight loss (see, for example, Non-Patent Document 2). In particular, it is found out that blood adiponectin level is lowered with the accumulation of visceral fat which strongly relates to the onset of obesity complications. It is also confirmed that, in a coronary artery disease, blood adiponectin level is lowered independently from the degree of obesity (see, for example, Non-Patent Document 3). In diabetes mellitus, blood adiponectin level is also lowered in proportion to the severity of diabetes mellitus (see, for example, Non-Patent Document 4). Moreover, it is reported that, in an experiment using monkeys, hypoadiponectinemia occurred before the onset of hyperinsulinemia and diabetes mellitus, which are indications of insulin resistance, in the course of the onset of obesity and type 2 diabetes mellitus induced by overeating and lack of exercise (see, for example, Non-Patent Document 5).

In nine primary hypoadiponectinemia cases caused by human gene mutations wherein isoleucine at the 164-th position of adiponectin was substituted by threonine, blood adiponectin levels amount to 25 to 30% of the normal level. Diabetes mellitus was observed in seven of these nine cases while borderline diabetes mellitus was observed in two cases. Among these nine cases, furthermore, patients in six cases suffered from coronary artery diseases such as myocardial infarction and angina pectoris. These data indicate that primary hypoadiponectinemia relates to insulin resistance and coronary diseases in humans too (see, for example, Non-Patent Document 6). In an analysis on adiponectin-knockout mice, diabetes mellitus with strong insulin resistance was observed when there animals were fed with a high-fat and high-sucrose diet for a short time (two weeks) and it was confirmed that this strong insulin resistance was ameliorated to the wild type level by supplying adiponectin to blood by using an adenovirus (see, for example, Non-Patent Document 7).

Patent Document 1 proposes an antiinflammatory agent and a monocyte growth inhibitor with the use of the effect of adiponectin of inhibiting the growth of monocyte lineage cells and B cells. Patent Document 2 proposes a liver fibrosis inhibitor with the use of the effect of adiponectin of promoting the growth of normal liver cells. As discussed above, it has been disclosed that the intake of adiponectin exerts an antiinflammatory effect and an effect of inhibiting liver fibrosis and contributes to the amelioration of the symptoms.

Also, it is reported that patients with human type 2 diabetes mellitus have a risk of myocardial infarction at a frequency thrice as high as healthy persons (see, for example, Non-Patent Document 8). Thus, it is presumed that lowering in blood adiponectin level brings about the onset of diabetes mellitus and, in its turn, induces myocardial infarction attack (see, for example, Non-Patent Document 1).

Recently, it is reported that a thiazolidine derivative, which is an agonist to peroxisome proliferator-activated receptor γ (PPARγ), elevates the blood adiponectin level of glucose intolerance patients (see, for example, Non-Patent Document 9), though there has been proposed no food, drink or composition that can be daily taken and elevate blood adiponectin level.

On the other hand, a high cholesterol level frequently results in the occurrence of coronary artery diseases such as myocardial infarction and angina pectoris. Cholesterol is transported by two lipoproteins called HDL (high-density lipoprotein) and LDL (low-density lipoprotein). HDL cholesterol withdraws cholesterol deposited in the vascular wall and transports it to the liver. On the other hand, LDL cholesterol transports cholesterol from the liver toward various tissues. As LDL cholesterol increases, therefore, the amount of cholesterol transported to every part of the body is elevated. As a result, there arises a tendency toward the promotion of the coronary diseases. Therefore, LDL cholesterol is called bad cholesterol. However, it has been clarified that some persons would not suffer from coronary artery diseases even though having high cholesterol level, while some have coronary artery diseases even though having low cholesterol level. The diameter of LDL cholesterol usually ranges from 26 nm to 27 nm. LDL cholesterol having a diameter less than 25.5 nm is called small dense LDL. Such small dense LDL is liable to be oxidized and the oxidized LDL causes a macrophage-foaming. Therefore, it appears that an increase in small LDL promotes coronary artery diseases.

Accordingly, it is meaningful in preventing diabetes mellitus and heart diseases such as coronary artery diseases or retarding the onset of these diseases to lower the small dense LDL level in blood by elevating blood adiponectin level and enlarging LDL particle size. Even in the case where these diseases arise, it is desired to take a countermeasure for the secondary prevention of the diseases. Since it is pointed out that the above-described diseases are caused by longstanding unhealthy life habits, it has been required to develop a food, a drink and a composition which can be regularly taken and remain highly safe even in prolonged intake. However, no safe food, drink or composition, which can lower the small dense LDL level in blood by elevating blood adiponectin level or enlarging LDL particle size, has been known hitherto.

Tea is a drink which has been favorably taken all over the world over 2,000 years. Various advantageous effects of tea have been known since ancient times. In recent years, it has been clarified that tea drinks such as oolong tea have various physiological functions. It has been reported that tea drinks have an antioxidant effect (see, for example, Non-Patent Document 10), an anti-obesity effect (see, for example, Non-Patent Document 11), an anti-stress effect (see, for example, Non-Patent Document 12) and so on. Depending on differences in production methods, tea is classified into non-fermented teas such as green tea and fermented teas involving semi-fermented teas such as oolong tea and completely fermented teas such as black tea, even though these teas are produced form leaves of the same plant *Camellia sinensis.* These teas differ in flavor from each other even in taking in usual manner. That is, components contained these tea extracts are largely different since they are produced by different production processes and steps.
Patent Document 1: JP-A-11-61095
Patent Document 2: Japanese Patent Application 2001-172882
Non-Patent Document 1: Tanpakushitsu Kakusan Koso, 2003, vol.47 (14), p.1896-1903
Non-Patent Document 2: Biochem. Biophys. Res. Commun., 1999, vol. 257, p.79-83
Non-Patent Document 3: Circulation, 1999, vol. 100, p. 2437-2476
Non-Patent Document 4: Arteriosclear Thromb Vasc. Biol., 2000, vol. 20, p.1595-1599
Non-Patent Document 5: Diabetes, 2001, vol. 50, p. 1126-1133
Non-Patent Document 6: Diabetes, 2002, vol. 51, p. 2325-2328
Non-Patent Document 7: Nature Med., 2000, vol. 8, p. 731-737
Non-Patent Document 8: Am. J. Med., 1998, vol. 105 (1A), p. 4S-14S
Non-Patent Document 9: Diabetes, 2001, vol. 50, p. 2094-2099
Non-Patent Document 10: J. Agric. Food Chem., 1999, vol. 47, p. 633-636
Non-Patent Document 11: Int. J. Obes. Relat. Metabl. Disord., 1999, vol. 23, p. 98-105
Non-Patent Document 12: Biosci. Biotechnol. Biochem., 2002, vol.66, p. 1955 - 1958

### DISCLOSURE OF THE INVENTION

### PROBLEMS THAT THE INVENTION IS TO SOLVE

A problem that the invention is to solve is to provide a composition capable of elevating blood adiponectin level and/or enlarging LDL particle size and thus lowering the concentration of small dense LDL in blood to thereby prevent diabetes mellitus and a heart disease such as a coronary artery disease or ameliorate the symptoms of these diseases, which originates in a natural material, shows a high safety without any side effect even in continuous intake over a long period of time, and is usable as a health food or drink. Another problem that the invention is to solve is to provide a composition comprising as the active ingredient a fermented tea extract which has an effect of elevating adiponectin level and/or an effect of enlarging LDL particle size.

### MEANS FOR SOLVING THE PROBLEMS

The inventors intensively conducted studies to search for a substance which is capable of elevating blood adiponectin level and/or enlarging LDL particle size and thus lowering the concentration of small dense LDL in blood to thereby prevent diabetes mellitus and a heart disease such as a coronary artery disease or ameliorate the symptoms of these diseases. As a result, they have found out that intake of a fermented tea extract makes it possible to elevate the blood adiponectin level of patients with coronary artery diseases including myocardial infarction and angina pectoris patients and enlarge LDL particle size to thereby lower the small dense LDL concentration in blood. In contrast, none of these effects is observed in green tea that is a fermented tea.

An extract of oolong tea that is a semi-fermented tea was prepared. Then, the oolong tea extract was given to 22 patients suffering from old myocardial infarction and stable angina for one month. One month after the initiation of the intake of the oolong tea extract, blood adiponectin levels and LDL particle sizes were compared with the data measured before the intake of the oolong tea extract. After giving the oolong tea extract for one month, the intake of the oolong tea extract was stopped for one month to examine the presence or absence of the efficacy of the oolong tea extract.

As a result, it was confirmed that the intake of the oolong tea extract caused not only significant elevation in blood adiponectin level but also significant enlargement in LDL particle size (see Example 2). As a subjective symptom, the patients expressed reduction in exertional fatigue. To prove the efficacy of the oolong tea extract, the intake of the oolong tea extract was stopped. One month after the wash out, it was confirmed that the blood adiponectin levels and the LDL particle sizes relapsed into the levels before the intake (see Example 2). Thus, it was confirmed that the elevation in blood adiponectin level and the enlargement in LDL particle size had been achieved by the intake of the oolong tea extract.

Accordingly, the invention provides a composition which comprises as the active ingredient a fermented tea extract and has an effect of elevating adiponectin level and/or an effect of enlarging LDL particle size. Namely, the invention provides a composition which comprises as the active ingredient a fermented tea extract and has an effect of elevating adiponectin level. Further, the invention provides a composition which comprises as the active ingredient a fermented tea extract and has an effect of enlarging LDL particle size. Furthermore, the invention provides a composition which comprises as the active ingredient a fermented tea extract and has an effect of elevating adiponectin level and an effect of enlarging LDL particle size.

The composition according to the invention may be a medicine employed by medical doctors in hospitals and so on or a marketed medicine. The composition according to the invention may be a health food, a functional food, food and drink including a food and a drink or an additive for such foods and drinks.

The term "fermented" as used in "fermented tea" herein means that, in the course of producing tea, tea leaves are not heated immediately after plucking so as to allow enzymes contained in the tea leaves to sustain the activities. For example, oolong tea, which is a semi-fermented tea, is generally obtained by a process comprising picking *Camellia sinensis* leaves (plucking), drying the tea leaves in the sun to vaporize moisture (sunshine withering), cutting the tea leaves to promote the fermentation (rocking green), heating and roasting the tea leaves in a pot to cease the fermentation (stir-fry green), kneading the tea leaves to facilitate extraction, and finally heating (drying). A completely fermented tea, which is typified by black tea, is generally obtained by a process comprising picking *Camellia sinensis* leaves and drying the tea leaves in the sun to vaporize moisture (sunshine withering), strongly kneading the tea leaves to promote the fermentation, fermenting the tea leaves (change color), and finally heating (drying). The semi-fermented tea typified by oolong tea differs from the completely fermented tea typified by black tea in that the fermentation is ceased by heating in the state where enzymes contained in tea leaves have not yet been inactivated in the former, while the fermentation is continued until the enzymes have been fully utilized and inactivated in the latter. However, both of these teas are produced from the same *Camellia sinensis* leaves via the fermentation step. Therefore, the term "fermented tea" as used herein means a tea produced by fermenting *Camellia sinensis* leaves. Namely, the term "fermented tea" as used herein includes any tea produced via the fermentation step such as a semi-fermented tea (for example, oolong tea) and completely fermented tea (for example, black tea).

Preferable examples of the fermented tea extract to be used in the invention include an oolong tea extract and a black tea extract.

The fermented tea extract to be used in the composition according to the invention can be obtained by, for example, extracting a fermented tea with an aqueous solvent. As the aqueous solvent to be used in the extraction, use may be made of either water alone or a solvent mixture prepared by blending water with one or more non-aqueous solvents selected from among a lower alcohol (for example, methanol, ethanol, etc.) and/or a polar solvent (for example, acetone, etc.) at an arbitrary mixing ratio. Considering the possibility of taking as food or drink, it is favorable to use water, ethanol or a mixture thereof from the viewpoint of safety. Although the ratio of the fermented tea to the extraction solvent is not specifically restricted in the extraction, it is favorable to use the extraction solvent in an amount from 2 to 1000 times by weight as much as the fermented tea. Taking the extraction performance and efficiency into consideration, it is still preferable to use the solvent from 5 to 100 times by weight as much as the fermented tea. The extraction temperature is not particularly restricted too. Namely, the extraction may be conducted at from room temperature to the boiling point of the extraction solvent. In particular, it is convenient from the viewpoint of working property to conduct the extraction at from room temperature to the boiling point of the extraction solvent under atmospheric pressure. The extraction time preferably ranges from 10 sec to 24 h. In extracting a fermented tea, it has been a practice to add sodium hydrogen carbonate to the extraction solvent or add various edible or medicinal components such as sodium hydrogen carbonate or sodium L-ascorbate to the extract so as to, for example, improve the preference for taking. These cases all fall within the scope of the invention, so long as such additives exert no undesirable effect on the fermented tea extract of the invention.

The fermented tea extract according to the invention may be in the form of a solution prepared by extracting the aqueous solvent as discussed above. Alternatively, it may be in the form of a solid obtained by drying (preferably freeze-drying) the extract solution. In one embodiment of the invention, therefore, the composition according to the invention having an effect of elevating adiponectin level and/or an effect of enlarging LDL particle size may contain, as the active ingredient, a fermented tea extract that is a solution obtained by extracting fermented tea leaves with water alone or a solvent mixture of water with a lower alcohol and/or a polar solvent. In another embodiment, the composition according to the invention having an effect of elevating adiponectin level and/or an effect of enlarging LDL particle size may contain, as the active ingredient, a fermented tea extract obtained by extracting fermented tea leaves with water alone or a solvent mixture of water with a lower alcohol and/or a polar solvent and further drying the thus obtained solution.

As the fermented tea extract of the invention, it is preferable to use one having a concentration of from 0.1% by weight to 40% by weight (Brix; solid matter content). In the case of oral intake, a tea drink commonly employed has a concentration of from 0.1% by weight to 1% by weight. Thus, a fermented tea extract having a concentration within the above range can be used as such as a drink, which is favorable from the viewpoint of preference. When the concentration of the fermented tea extract is higher than the level commonly employed as a drink, it sometimes strongly tastes bitter when taken as such. In such a case, it is preferable to use the fermented tea extract in the form of an extract or a freeze-dried powder, or process it into a tablet or a capsule and use in a health food or drink.

In the case of using in a health food or drink, the fermented tea extract of the invention can be added to, for example, a dry food, a supplement, a refreshing drink, mineral water, an alcoholic beverage and so on, though the invention is not restricted thereto.

In the case of using the fermented tea extract of the invention in a health food or drink or as a food additive, it may be processed into a preparation. The preparation may be either a solid preparation or a liquid one. As examples of the preparation, there can be enumerated a powder, a tablet, a pill, a capsule, a granule, a suspension, an emulsion and so on. The preparation of the invention may contain fillers acceptable in preparations. As the fillers, use can be made of a diluent, a perfume, a stabilizer, a lubricant for suspensions, a binder, a preservative, a disintegrating agent for tablets and so on. Either one of these fillers or a combination thereof can be used.

In the case of using the fermented tea extract of the invention in a health food or drink or as a food additive, it is desirable to process or blend the fermented tea extract in such a manner as to give a daily intake of from about 0.1 g to about 3 g. However, the fermented tea extract of the invention may be processed or blended to give a daily intake exceeding the level as defined above, since it is produced form tea that has been taken everyday and has a high safety without showing any side effect even though it is taken in an amount exceeding the above level.

To elevate blood adiponectin level and/or enlarge LDL particle size in blood to thereby ameliorate the symptoms of diabetes and heart diseases such as coronary diseases, the composition according to the invention may be administered/taken within a relatively short time (for example, a single administration to one month) or continuously over a long period of time, i.e., longer than one month. Also, it may be administered/taken continuously over a long time to prevent the diseases as described above.

The effects of the composition according to the invention can be evaluated based on patients' own scent of the amelioration in the subjective symptoms of diabetes mellitus and heart diseases such as coronary artery diseases caused by the administration/intake of the composition according to the invention, medical findings showing the relief in the symptoms of the above diseases, prevention of the onset of these diseases and so on. If necessary, the effects can be also evaluated by measuring blood adiponectin level and/or the LDL particle size in blood.

The effect of elevating adiponectin level is determined by comparing the blood adiponectin level before the intake of the fermented tea extract of the invention and the blood adiponectin level after the intake thereof. In a typical case, blood adiponectin level is measured by an enzyme immunoassay method (an adiponectin measurement kit, OTSUKA PHARMACEUTICAL Co., Ltd.) with the use of the plasma of a subject. In the case where an increase is observed in the comparison of the blood adiponectin level before the intake of the fermented tea extract of the invention and the blood adiponectin level after the intake thereof, it is judged that the fermented tea extract of the invention has an effect of elevating adiponectin level.

The effect of enlarging LDL particle size is determined by comparing the LDL particle size before the intake of the fermented tea extract of the invention and the LDL particle size after the intake thereof. In a typical case, LDL particle size in blood is measured by electrophoresing the serum of a subject on a polyacrylamide gel disc (LIPOPHOR, JOKO Co.) and analyzing the electrophoteric pattern thus obtained by densitometry (CLINISCAN 2, HELENA LABORATORIES).
In the case where an increase is observed in the comparison of the LDL particle size before the intake of the fermented tea extract of the invention and the LDL particle size after the intake thereof, it is judged that the fermented tea extract of the invention has an effect of enlarging LDL particle size, i.e., being capable of lowering the small dense LDL concentration in blood.

Next, the invention will be described in greater detail by reference to Examples.

### ADVANTAGE OF THE INVENTION

It is highly useful to take the fermented tea extract, since the fermented tea extract can significantly enlarge LDL particle size and significantly elevate blood adiponectin level to thereby prevent diabetes mellitus and heart diseases such as coronary artery diseases or ameliorate symptoms of these diseases.

Oolong tea is a semi-fermented tea originating in China. It is produced mainly in Fujian and Guang Dong Provinces in China and Taiwan. After the release of canned oolong tea in Japan, these products are highly accepted because of the convenience. At the same time, oolong tea matches consumers' health-consciousness with preference to sugar-free drinks. Under these circumstances, oolong tea has spread rapidly and widely taken in these days. Although oolong tea that is a tea drink is taken in a large amount everyday over a long period of time, no adverse effect thereof has been reported hitherto. Thus, it is a highly safe drink. Accordingly, the fermented tea extract used in the invention has an extremely high safety particularly in continuous intake over a long time. Moreover, it is a fermented tea that is familiar to sick persons in daily life and has been usually taken. With these factors in the background, the fermented tea extract can be safely and positively taken without any unpleasantness or anxiety. For preventive purposes, it can be also taken without feeling uncomfortable.

### EXAMPLES

### Example 1: Production of fermented tea extract

1000 ml of deionized water was added to 60 g of oolong tea leaves, i.e., a semi-fermented tea, and extraction was conducted at 95°C for 10 min. The mixture was filtered and the filtrate thus obtained was centrifuged. The obtained supernatant was heat-sterilized (95°C, 6 sec) and then freeze-dried to give 15 g of a fermented tea extract. Table 1 shows the results of the analysis on the main components in the whole fermented tea extract liquid.

**[Table 1]**

| Table 1: Contents of main components of oolong tea extract | |
|---|---|
| Component | Content (mg) |
| Gallic acid | 52.5 |
| Epigallocatechin | 507 |
| Catechin | 27.2 |
| Caffeine | 551 |
| Epicatechin | 151 |
| Epigallocatechin gallate | 454 |
| Gallocatechin gallate | 38.1 |
| Epicatechin gallate | 129 |
| Catechin gallate | 18.2 |
| Other polyphenols | 528 |

### Example 2: Effect of elevating adiponectin level in blood by taking oolong tea extract

After obtaining sufficient informed consent, 22 human subjects including 12 suffering from old myocardial infarction and 10 suffering from stable angina pectoris (average age: 64.3±7.2 years, BMI: 23±4, 17 males and 5 females) voluntarily participated in the test. Coronary risk factors of these 22 subjects were as follows; seven hypertension patients, nine diabetics, 12 hypercholesterolemia patients, seven obese persons and five smokers. The subjects individually took required medicines during the test period.

As the fermented tea extract, a solution of 1.5 g of the oolong extract obtained in Example 1 dissolved in 1000 ml of deionized water was taken everyday in several portions before going to bed. After taking for one month, blood adiponectin level, total cholesterol, LDL cholesterol, HDL cholesterol, triglyceride and LDL particle size were measured and compared with the data before the intake. Also, glucose level and hemoglobin Alc were measured in some (nine) of the subjects. After stopping the intake of the oolong tea extract for one month, the blood was collected again and the effects after wash out were confirmed. The blood adiponectin level was measured by an enzyme immunoassay method using an antibody constructed with the use of recombinant human adiponectin (OTSUKA PHARMACEUTICAL Co., Ltd.), while the LDL particle size was measured by the disc electrophoresis method by using polyacrylamide (JAMA, 260, 1917-1921, 1988).

Table 2 shows the results. It was clarified that the blood adiponectin level was significantly elevated from 6.26±3.26 µg/ml before the intake of the oolong tea extract to 6.88±3.28 µg/ml after the intake of the oolong tea extract (p<0.05). After wash out, the adiponectin level relapsed into 6.28±3.28 µg/ml, i.e., being almost the same level as before the intake of the oolong tea extract.

It was clarified that the LDL particle size was significantly enlarged from 25.02±0.67 nm before the intake of the oolong tea extract to 25.31±0.60 nm after the intake of the oolong tea extract (p<0.01). After wash out, the LDL particle size relapsed into 25.11±0.70 nm, i.e. being almost the same level as before the intake of the oolong tea extract.

The total cholesterol was significantly lowered from 209±30 mg/dl before the intake of the oolong tea extract to 197±25 mg/dl after the intake of the oolong tea extract (p<0.01). After wash out, the total cholesterol was 200±29 mg/dl, i.e., being still significantly lower than the level before the intake of the oolong tea extract (p<0.05).

The blood glucose level measured in a part of the subjects (9) showed a tendency toward lowering though not being significant. The hemoglobin Alc became significantly lower after taking the oolong tea extract for one month (p<0.05).

It was found out that, by taking the oolong tea extract for one month, the patients expressed reduction in exertional fatigue as a subjective symptom.

**[Table 2]**

| Table 2: Effect of ameliorating blood adiponectin level of human coronary artery disease patients by taking oolong tea extract | | | |
|---|---|---|---|
| (n=22) | Before intake | After intake | After wash out |
| Adiponectin (µg/dl) | 6.26±3.26 | 6.88±3.28* | 6.28±3.28# |
| Total cholesterol (mg/dl) | 209±30 | 197±25** | 200±29* |
| LDL cholesterol (mg/dl) | 123±25 | 117±24 | 115±27 |
| HDL cholesterol (mg/dl) | 52±14 | 50±13 | 50±12 |
| LDL particle size (nm) | 25.02±0.67 | 25.31±0.60** | 25.11±0.70# |

| (n=9) | | | |
|---|---|---|---|
| Glucose (mg/dl) | 173±69 | 156±53 | 163±49 |
| Hemoglobin Alc (%) | 7.23±4.45 | 6.99±4.30* | 7.12±4.21 |

| | | | |
|---|---|---|---|
| *: p<0.05 Compared with the level before the intake. **: p<0.01 Compared with the level before the intake. #: p<0.05 Compared with the level after the intake. | | | |

### Example 3: Powder and Granules

To 10 g of the oolong tea extract produced in Example 1, 53 g of lactose and 16 g of corn starch were added. The obtained mixture was wet granulated with a corn starch binder to give powder and granules.

### Example 4: Production of tablets

The powder and granules produced in Example 3 were blended with 1 g of sucrose fatty acid ester and the obtained mixture was tabletted to give tablets each weighing 300 mg.

## Claims

1. A composition which comprises as the active ingredient a fermented tea extract and has an effect of elevating adiponectin level and/or an effect of enlarging LDL particle size.

2. A composition as claimed in claim 1 which is a medicine.

3. A composition as claimed in claim 1 which is a food or drink or to be added to a food or drink.

4. A composition as claimed in any one of claims 1 to 3 which comprises as the active ingredient a fermented tea extract and has an effect of elevating adiponectin level and an effect of enlarging LDL particle size.

5. A composition as claimed in any one of claims 1 to 4 wherein the fermented tea extract is an oolong tea extract or a black tea extract.

6. A composition as claimed in any one of claims 1 to 5 which comprises as the active ingredient a fermented tea extract obtained by extracting fermented tea leaves with water alone or a mixture of water with a lower alcohol and/or a polar solvent.

7. A composition as claimed in claim 6 which comprises as the active ingredient a fermented tea extract obtained by extracting fermented tea leaves with water alone or a mixture of water with a lower alcohol and/or a polar solvent and then drying.
